# EUROPEAN PATENT SPECIFICATION

(11) **EP 1 237 587 B1**
(45) Date of publication and mention of the grant of the patent: **10.03.2004**
(21) Application number: 00990126.5
(22) Date of filing: 04.12.2000
(51) Int. Cl.: A61L 29/08, A61L 31/10, A61L 29/16, A61L 31/16

(54) **WIRE, TUBE OR CATHETER WITH HYDROPHILIC COATING**
DRAHT, SCHLAUCH ODER KATHETER MIT FEUCHTHALTEMITTELBESCHICHTUNG
FIL METALLIQUE, TUBE OU CATHETER COMPRENANT UN REVETEMENT HYDROPHILE

(30) Priority: 03.12.1999 EP 99204138
(43) Date of publication of application: 11.09.2002
(73) Proprietor: MCTec B.V., 5928 PG Venlo (NL)
(72) Inventor: KOOLE, Levinus, Hendrik, NL-6271 EC Gulpen (NL); HANSSEN, Johannes, Hendrikus, Leonardus, NL-6577 JL Erlecom (NL)
(74) Representative: Prins, Adrianus Willem
(86) International application number: PCT/NL2000/000888
(87) International publication number: WO 2001/039811

(56) References cited:
- EP-A- 0 945 148
- US-A- 5 605 696
- US-A- 5 607 417

## Description

The invention relates to a wire, tube or catheter coated with at least one layer of a hydrophilic biocompatible material comprising a pharmacologically active compound, to a medical device for the controlled release or (on-site) delivery of pharmacologically active compounds, and to a method of preparation thereof.

Metallic wires and tubes, metallic coils, and polymeric catheters are widely used in clinical practice, especially in non-invasive diagnostics or therapy. Important examples are found in non-invasive cardiology. In many cases, the wires, coils, or catheters are covered by a thin coating, which usually consists of poly(tetrafluoro-ethylene), or of a lubricious hydrophilic polymeric material. Products with a hydrophilic coating are designed to feature excellent lubricity and a high level of biocompatibility, in the sense that they have low thrombogenicity, little or no propensity to activate contacting blood platelets, and do not invoke irritation of the surrounding tissues.

The use of wires, coils and catheters in the cardiovascular system is common practice, e.g. during the treatment or coronary disease through PTCA (percutaneous transluminary coronary angioplasty). One of the drawbacks associated with the use of wires, coils and catheters which are used in the blood vessel system it is still necessary to administer an anticoagulant drug to the patient. This anticoagulant drug (usually heparin) prevents coagulation of blood as a result of the contact of the blood with the artificial surface of the wire, coil, or catheter. The administration of the anticoagulant is normally performed intravenous. Due to the a-selective administration of the anticoagulants this method is associated with a significant risk for bleeding. It is extremely difficult to cope with bleeding of heparinized blood, especially when the bleeding occurs inside the body. A very delicate balance must be maintained during the administration of anticoagulant drugs during non-invasive manipulations in the cardiovascular system: administration of too much heparin poses the patient at a high bleeding risk, and administration of too little heparin may result in coagulation leading to the formation of emboli and blood clots.

It is a goal of this invention to come to a delivery system for pharmacologically active compounds whereby the controlled release and/or the (site-specific) delivery of the active compound are enabled.

It is a further goal of the invention to find an efficient solution to the problem of adequate administration of anticoagulant agent prior to, during, and after the use of wires, coils, or catheters in the cardiovascular system.

It has now been found that the application of a coating of swellable hydrophilic biocompatible polymeric material impregnated with a pharmacologically active compounds to a wire, tube or catheter results in product that provides for a system for the administration of these active agents. By incorporation of the active agent in the swellable polymer, the active agent will be released upon swelling of the polymer.

Accordingly, the invention relates to a wire, tube or catheter coated with at least one layer of a hydrophilic biocompatible material containing at least one pharmacologically active compound.

In a specific embodiment the invention relates to such wires, tubes or catheter having a biocompatible coating, which wire, tube or catheter is suitable for controlled drug release.

The wires, coils, and also catheters according to the invention have a hydrophilic polymeric layer or coating in such a way that the pharmacologically active compound or drug is impregnated in the polymeric coating.

These products are basically intended for temporary use, which is typically in the range of 5 - 180 minutes, but the use of these products for the controlled release or delivery of drugs over a longer period (days, weeks) is also within the scope of the invention. The coated and drug loaded product according to the invention is sterilised prior to its use. The wire, coil, or catheter is introduced into the body according to the normal procedure. This means that a small incision is made, and that the tip of the wire, coil or catheter is forwarded to the desired location in the body. The unique feature of this invention is that the hydrophilic coating on the product will immediately start to swell and to release the drug upon the contact with an aqueous environment.

The aqueous environment can be provided by the vascular system, the urinary tract, or by placement of (part of) the wire, coil, or catheter in other places in the body for instance intra-abdominal or intra-articular, intracapsular or intra-ocular. For instance, if the product is inserted into the blood vessel system, the contact with the blood in the vascular system represents, the contact with the aqueous environment. Accordingly the hydrophilic polymer will swell and subsequently release the drug.

The swelling of the coating, as well as the release of the drug into the bloodstream or other parts of the body are essentially diffusion processes. Without being bound by any statement it is thought that the swelling of the biocompatible material is caused by the absorption of water by the material. This is also a criterion for the selection of the hydrophilic component of the biocompatible material.

The kinetics of swelling and drug release can be controlled via synthesis of the polymeric coating: a more hydrophilic coating will show fast swelling, and fast concomitant release of the drug. Likewise by employing a less hydrophilic coating the swelling of the coating will be less resulting in a slower release of the drug. The concentration of the drug in the polymeric coating also determines the amount of drug that is released during the use of the wire, coil or catheter. The amount of the drug that is released can further be controlled by the application of an additional layer of the same hydrophilic biocompatible swelling material which additional layer does not comprise the drug or of the application of a biocompatible and swellable material or a slowly dissolving material to further tune the release kinetics of the drug.

Several applications of this novel strategy for controlled local drug delivery are foreseen, apart from the application relating to controlled release of heparin or an other anticoagulant agent from the surface of coated wires, tubes or catheters. Examples of the other applications are: (i) local administration of a cytostatic agent from the extreme coated part (tip) of a coil, wire tube or catheter which is forwarded through the vascular tree towards a solid tumour; (ii) slow release of one or more cytostatic agents from the surface of a wire, coil, or catheters in the blood stream; (iii), slow release of antibiotic agents from the surface of a wire, coil or catheter which is inserted in the urinary tract; (iv) manufacture of catheters, drains, or other tubing which featuring slow release of an antibiotic agent, in such a way that the risk of infection of the catheter is reduced; (v) slow release of an antibiotic agent (e.g. gentamicin) from the surface of coated wires or mesh structures in order to combat infections, e.g. biomaterial-associated infections.

The wire, tube or catheter according to the invention is made from a metal or an alloy, or a polymeric material or a combination thereof. Examples of suitable metals and alloys are stainless steel, tantalum, platinum, gold and shape-memory alloys such as nitinol.

The form of the wire, tube or catheter is, in general, not critical. However, when the wire, tube or catheter is in the form of a spring or a coil, this is considered advantageous as it allows for a relative large surface in a small volume. This design allows for a more precise control of the delivery of a suitable amount of drugs.

The polymeric coating is applied in such manner to the wire, tube or catheter that the material provides an adherent matrix, suitable for the incorporation of drugs on the wire, tube or catheter. The polymer coating is applied to the surface of the product in one of the final steps of its manufacturing, but prior to sterilisation. The polymeric biomaterial can be applied as a solution in a volatile organic solvent, via a spray process, a dip-coating process, or otherwise. This may be followed by a treatment of the coated product at elevated temperature and/or vacuum, in order to facilitate evaporation of residual solvent molecules, and/or in order to achieve firm attachment of the polymer coating to the metallic surface The application of a primer coating, which is sandwiched between the metallic surface and the polymeric biomaterial may be advantageous. The copolymers as described above can be dissolved in a volatile organic solvent, and can be applied to the product via a dip-coating procedure or via a spray process. Other processes resulting in a suitable coating on the metal may also be used. The final hydrophilic polymeric coating provides an adherent matrix in the dry state, in the fully hydrated wet state, as well as in all intermediate states of partial hydration which are passed during the process of swelling and release of the drug in situ. Swelling of the coating polymer facilitates the diffusion of drug into the blood stream, while no dissolution of the polymeric material occurs.

The invention according to another aspect also comprises the hydrophilic biocompatible material and the synthesis of this biomaterial out of which the drug-carrying coating is constructed.

The coating on the wire, tube or catheter in general comprises a composition that is swellable. A swellable polymer is obtained by preparation of a copolymer of a hydrophilic component and a hydrophobic component. The hydrophilic component will allow the polymeric material to swell whereas the hydrophobic material prevents the dissolution of the polymeric material. In a preferred embodiment, the hydrophilic biocompatible polymeric material comprises a hydrophilic component and a hydrophobic component.

In an embodiment of the invention, the polymeric coating material is a copolymer of a hydrophobic reactive monomer, and a hydrophilic reactive monomer. A reactive monomer that is chemically reactive in such a manner that it can participate in free radical polymerisation reactions.

The desired swelling characteristics and the release kinetics of the drug are influenced by the ratio of hydrophilic and hydrophobic units in the polymeric material. The incorporation of a relative large amount of hydrophilic units results in a polymeric material that is very swellable and the contact with an aqueous environment will result in a quicker release of the drug than in the case where a relative low amount of hydrophilic units is incorporated in the polymeric material. The release and/or the delivery of the drug is thus controlled by the ratio of the hydrophilic and hydrophobic component. Accordingly, in a preferred embodiment the ratio of the hydrophilic and hydrophobic component is adjusted to control the controlled release and/or delivery of the pharmacologically active compound.

The ratio of the hydrophilic component to the hydrophobic component ranges from 0.1 to 100, preferably from 0.2 to 75, more preferably from 0.3 to 50. But also ratios of 1 to 10.2 to 20.3 to 30 are also possible.

The hydrophobic monomer is selected from hydrophobic acrylates and methacrylates, preferably N-butylmethacrylate, but may also be selected from other hydrophobic (meth)acrylates such as for instance disclosed in NL-A-1001746.

The hydrophilic monomer is chemically reactive, in such a way that it can participate in free-radical polymerisation reactions. A further requirement is that the hydrophilic monomer is soluble in water under ambient conditions.

The hydrophilic reactive monomer is N-vinylpyrrollidinone.

The reactive monomers are subjected to a polymerisation reaction, and the product is dissolved in an organic solvent, such as N-methylpyrrollidinone (NMP).

The biocompatible polymer according to the invention has a molecular weight of 10.000 to 100.000, preferably in the range 100.000 to 500.000.

Subsequently a solution of the pharmacologically active agent is added under continuous stirring. The resulting composition is used in the coating process. The polymerisation reaction and coating process are disclosed in NL-A-1001746.

The biocompatible swellable material of the invention can be modified by adding crosslinkers to the intial mixture of monomers, by this the swelling behaviour and drug release kinetics can be further modified. Suitable crosslinkers are, but are not limited to: (i) tetraethyleneglycoldimethacrylate; (ii), ethyleneglycoldimethacrylate; (iii), ethyleneglycoldiacrylate.

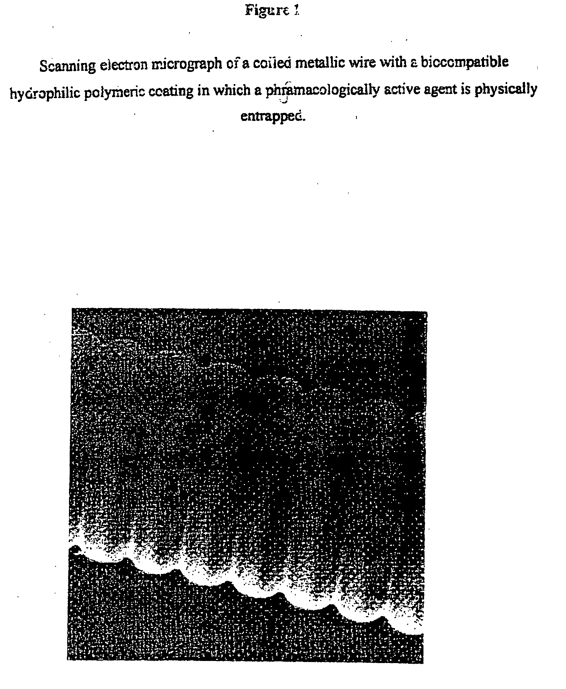

The resulting wires have a smooth uniform thin polymeric coating which contains the drug in impregnated form. The wires can be coiled without the occurrence of cracking of the coating. The resulting coils show uncompromised lubricity and biocompatibility. Such coils have been tested with regard to controlled drug release in a series of experiments in vitro. Figure 1 shows a representative example of a coiled metallic wire with a hydrophilic coating which contains a pharmacologically active drug (heparin); (image obtained with scanning electron microscopy).

### Description of the figures:

Figure 1: Scanning electron micrograph of a coiled metallic wire with a biocompatible hydrophilic polymeric coating in which a pharmacologically active agent is physically entrapped.
Figure 2: Cumulative release curves of rhodamine from three different coils.
Figure 3: Release of heparin from a metallic coil with a heparin charged hydrophilic polymeric coating.

The invention will now be further elucidated by the following examples.

### Example 1.

Experiments on controlled release of a model drug from three different coatings on metallic wires were performed. Rhodamine was chosen as the dye. Rhodamine (C₂₈H₃₁ClN₂O₃, M = 479.0) is a water-soluble dye (CI 45170) having maximum UV extinction at lambda of 528 nm and showing intense fluorescence. Rhodamine is also soluble in NMP. The dye was added to the solution of the copolymer in NMP prior to the coating procedure in a ratio of 1:100 (wt rhodamine: wt. copolymer). The coatings were applied to metallic wires, according to the method disclosed in NL-A-1001746. The three wires differed merely with respect to the hydrophilicity of the coating (see Table 1). The wires were coiled on a mandrill with a diameter of 2.0 mm.

**Table 1.**

| Material | Composition, expressed as molar ratio NVP : alkylmethacrylate |
|---|---|
| 75/25 | 3 : 1 |
| 90/10 | 9 : 1 |
| 95/5 | 19 : 1 |

Relatively short coils were prepared (typical length = 20 cm). A total weight of 15.0 g (which corresponds to a length of 78.9 m) was immersed in 750 mL of distilled water. The concentration of rhodamine, dissolved in the buffer, was determined spectrophotometrically as a function of time.

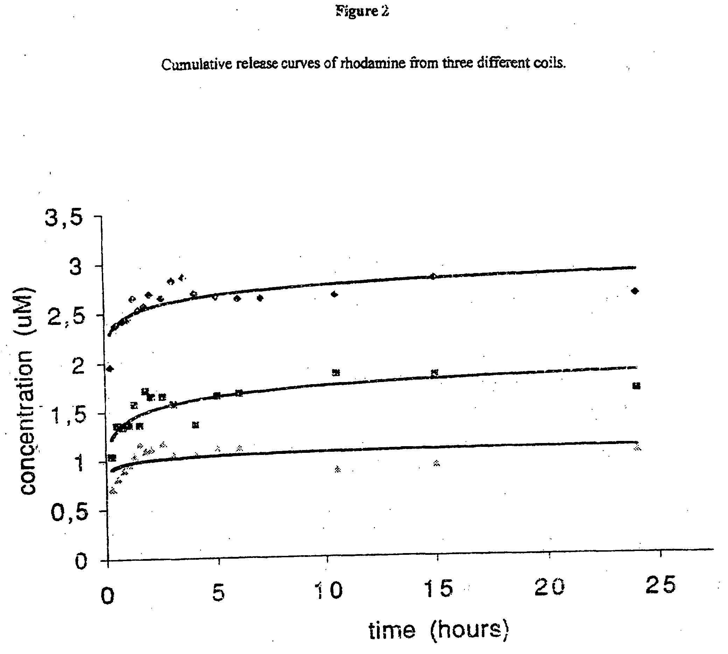

Figure 2 shows the cumulative release curves measure for the three different coils with a rhodamine-containing coating. It is clear that the most hydrophilic coating (95/5) corresponds to the highest concentration of rhodamine in solution, as was expected a priori. The release kinetics are not significantly influenced by the coating; the rhodamine concentration profiles show a plateau which is reached approximately 2 hours after immersion. In all three cases it was noted visually that there was still rhodamine entrapped in the coating on the coils at this stage. Therefore, the release experiments were continued during several weeks, but the rhodamine concentration in the buffer, as well as the colour of the coils, remained essentially unchanged. Mass balances revealed that a relatively large fraction of the rhodamine remained entrapped in the coating. The calculated amounts of released rhodamine are: 5.1 % for the 80/20 coating, 8.0 % for the 90/10 coating, and 13.3 % for the 95/5 coating.

### Comparative Example 2.

Four layers of polymeric coating were deposited on a stainless steel wire with a diameter of 178 micrometer and a length of approximately 1000 meters. The first two layers consisted of poly(ethersulfone) as a primer coating, according to prior art (J.H.L. Hanssen, L.H. Koole, "Guidewire for medical applications.", International Patent Nr. 1001746, November 27, 1995). The third and fourth layer (i.e. the outermost layers) were deposited from an emulsion consisting of: N-methylpyrrollidinone (200 milliliter), the copolymer 90/10 (vide supra), water (20 milliliter), and heparin (1.00 gram). This emulsion was stirred continuously in order to prevent precipitation of heparin due to phase separation.

The resulting coated wires were coiled around a mandrill, and prototype guidewires were manufactured. It was observed that the coiling procedure did not lead to the formation of cracks of any other damaging of the polymeric coating. Further these guidewires exhibit a similar level of lubricity as compared to their counterparts which do not contain heparin in the outermost layer or layers.

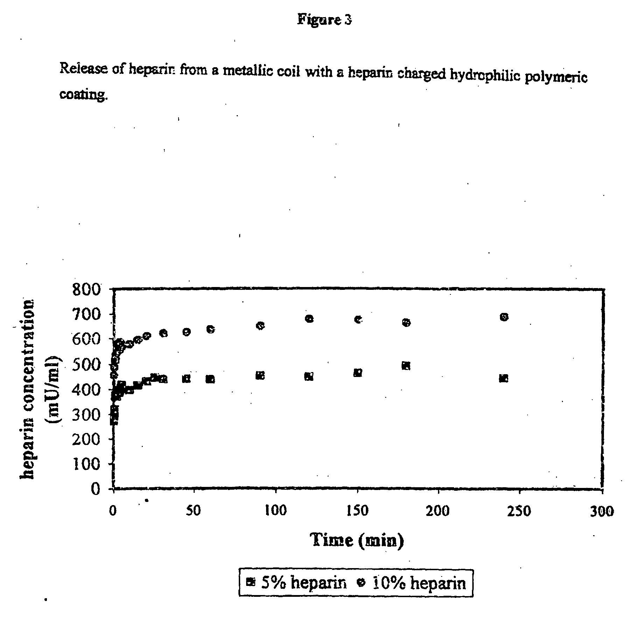

Pieces of the coil (approximately 20 grams in total), were immersed in an aqueous buffer solution (phosphate buffered sahne, pH 7.4) (200 millilitre) which was maintained at 37 °C. Samples of 1 millilitre were withdrawn from the buffer solution at regular time points. The concentration of heparin in these samples was determined using well-established techniques. The results of these experiments are shown in Figure 3. It is clear that slow release of heparin occurs from the surface of the wire. The hydrophilic polymeric coating serves as a temporary depot for the heparin. The anticoagulant activity of the heparin after the release is not influenced as a result of the coating and redissolution. In this example, the heparin is released over a time period of approximately 2 hours. Initially, the heparin release is relatively fast. The release process gradually levels off and came to a stop around 2 hours after the immersion of the coils in the buffer solution.

In general the kinetics of the release of the drug can be controlled via different strategies, such as: (i) change of the hydrophilicity of the polymeric coating (a more hydrophobic coating will lead to slower swelling in an aqueous environment, and to a slower release of the impregnated drug); (ii) increase or decrease of the amount of the drug which is immersed in the coating; (iii) application of an additional hydrophilic coating not containing any pharmacologically active compounds as a controlled release coating. It is clear that each of these strategies have limitations, e.g. increasing the hydrophilicity of the coating may lead to detachment of the polymer coating from the wire. This may be combated by the use of polymers that form an adhesive layer (primer coating) between the wire and the hydrophilic coating, thus enhancing the adhesion of the hydrophilic coating. Combination of the different strategies will tune the kinetics of the release process to the desired application, for instance for use during interventional cardiology.

It is inferred from these observations that it is possible to realise controlled release of heparin, or any other anticoagulant agent, or another substance with pharmacological activity, from the surface of a polymer-coated wire, coil, or tube (catheter), where the base material can be metallic or polymeric. The moment of introduction of the wire, coil or tube in the circulation marks the start of the drug release, since swelling of the coasting essentially liberates entrapped heparin molecules or one or more of the other pharmacologically active agents. This strategy facilitates the application of wires, coils or tubes for temporary use in the human circulation, as is the case during, for instance, routine operations in interventional cardiology. It must be noted that release of heparin over a time interval of 2 hours (viz. Figure 3) is close to what is desired for applications in interventional cardiology.

In the case of heparin or another anticoagulant agent, it is important that such controlled release occurs exactly where the drug is needed, i.e., at the interface of the wire, coil, or tube (an artificial surface) and the blood stream. The data in Figure 3 imply that the local concentration of heparin in the vicinity of the artificial surface is sufficiently high to prevent coagulation.

The most important implication of these results is that heparin-loaded coils, wires or tubes, according to this invention, can be used in combination with reduced systemic heparinisation of the patient. This, in turn, has the important advantage that the risk of bleeding complications is reduced. This is considered a very important beneficial effect of the present invention.

### Example 3.

A further application of this invention relates to the impregnation of an antibiotic agent in the hydrophilic polymeric coating on wires, coils or tubes. Such a product can be used to combat biomaterial-associated infections, or to reduce the risk of their occurrence. Important examples comprise, but are not limited to: (i), infections occurring during the use of indwelling catheters, and (ii), infections occurring after placement of a hip prosthesis or another orthopaedic prosthesis. With respect to this last example, it is common practice to eliminate the infection (e.g., in the femoral cavity) through the use of gentamicin-charged polymeric beads which are connected via a metallic string. This is an adequate solution, which, however, has the drawback that the removal of the beads on the string can generate a severe new wound in the femoral cavity, with a serious risk for re-infection. An alternative for the use of gentamicin beads is the use of a mesh of a metallic wire with a gentamicin-charged hydrophilic polymeric coating. Such a construct can be made according to the invention such that: (i), release of gentamicin or other antibiotics occurs over a predetermined time interval (e.g. two weeks), (ii), the construct is slippery and easily removable from the wound without the generation of a new wound.

### Example 4.

Administration of drugs to the vitreous body of the eye, or to any other part of the eye, poses formidable technical challenges, especially when perforation of the sclera has to be prevented. Yet, administration of drugs to the vitreous body or to other compartments of the eye is extremely important, (e.g. administration of gancyclovir for the combat of CMV retinitis). Short wires, or coils according to this invention are used as temporary vehicles to achieve improved administration of drugs to the eye. A drug-charged coil is placed in the vicinity of the sclera in a parallel fashion, or inside the scleral tissue, but in such manner that no perforation of the sclera occurs. Then, release of the drug from the hydrophilic coating is realised, and diffusion of the drug into the eye occurs. The wire or coil can be removed after it is exhausted. The exhausted wire or coil is replaced with a new, charged wire or coil.

## Claims

1. Wire, tube or catheter coated with at least one layer of a hydrophilic biocompatible material containing at least one pharmacologically active compound, wherein the hydrophilic biocompatible material is a polymeric coating material comprising a hydrophilic monomer which can participate in free-radical polymerisation reactions and which is soluble in water under ambient conditions, said hydrophilic monomer being N-vinylpyrrolidinone.

2. Wire, tube or catheter according to claim 1, wherein the wire, tube or catheter is from a metal, alloy, or polymeric material or a combination thereof.

3. Wire, tube or catheter according to claim 1 or 2, wherein the wire tube is in the form of a coil or a spiral.

4. Wire, tube or catheter according to claims 1-3, wherein the hydrophilic biocompatible material is a copolymer of a hydrophobic reactive monomer and a hydrophilic reactive monomer.

5. Wire, tube or catheter according to claim 4, wherein the ratio of the hydrophilic component and the hydrophobic component is adjusted to control the controlled release and/or delivery of the pharmacologically active compound.

6. Wire, tube or catheter according to claims 4 or 5, wherein the ratio of the hydrophilic component and the hydrophobic component of the hydrophilic biocompatible material is from 0.1 to 100.

7. Wire, tube or catheter according to claims 4-6, wherein the hydrophobic component is selected from hydrophobic acrylates and methacrylates, preferably N-butylmethacrylate.

8. Wire, tube or catheter according to claims 1-7, wherein the hydrophilic biocompatible material further comprises a cross-linker.

9. Wire, tube or catheter according to claims 1-8, wherein the biocompatible material is additional coated with a (partly) soluble or swellable material to further control the release and/or delivery of the pharmacologically active compound.

10. Wire, tube or catheter according to claims 1-9, wherein the pharmacologically active compound is selected from the group of heparin, anti-coagulent factors, cytostatic agent and anti-biotics.

11. Medical device for the controlled release and/or (on-site) delivery of pharmacologically active compounds comprising a coated wire, tube or catheter according to claims 1-10.

12. Method for the preparation of a medical device comprising applying at least one layer of composition comprising a hydrophilic biocompatible materials according to claim 1-11 and a pharmacologically active compound to at least part of a wire, tube or catheter.

## Patentansprüche

1. Draht, Schlauch oder Katheter, welcher mit wenigstens einer Schicht aus einem hydrophilen biologisch verträglichen Material beschichtet ist, das mindestens eine pharmakologisch wirksame Verbindung enthält und ein polymeres Beschichtungsmaterial ist, das ein hydrophiles Monomer umfasst, das an radikalischen Polymerisationsreaktionen teilnehmen kann, unter Umgebungsbedingungen wasserlöslich ist und N-Vinylpyrrolidinon ist.

2. Draht, Schlauch oder Katheter nach Anspruch 1, der aus einem Metall, einer Legierung bzw. einem polymeren Material oder einer Kombination davon besteht.

3. Draht, Schlauch oder Katheter nach Anspruch 1 oder 2, wobei der Draht oder Schlauch die Form einer Wicklung oder Spirale hat.

4. Draht, Schlauch oder Katheter nach den Ansprüchen 1 bis 3, wobei das hydrophile biologisch verträgliche Material ein Copolymer aus einem hydrophoben reaktiven Monomer und einem hydrophilen reaktiven Monomer ist.

5. Draht, Schlauch oder Katheter nach Anspruch 4, wobei das Verhältnis von hydrophiler zu hydrophober Komponente so eingestellt wird, dass die kontrollierte Freisetzung und/oder Abgabe der pharmakologisch wirksamen Verbindung gesteuert wird.

6. Draht, Schlauch oder Katheter nach Anspruch 4 oder 5, wobei das Verhältnis von hydrophiler zu hydrophober Komponente des hydrophilen biologisch verträglichen Materials 0,1 bis 100 beträgt.

7. Draht, Schlauch oder Katheter nach den Ansprüchen 4 bis 6, wobei die hydrophobe Komponente aus hydrophoben Acrylaten und Methacrylaten und vorzugsweise N-Butylmethacrylat ausgewählt ist.

8. Draht, Schlauch oder Katheter nach den Ansprüchen 1 bis 7, wobei das hydrophile biologisch verträgliche Material außerdem ein Vernetzungsmittel enthält.

9. Draht, Schlauch oder Katheter nach den Ansprüchen 1 bis 8, wobei das biologisch verträgliche Material außerdem mit einem (teilweise) löslichen oder quellfähigen Material beschichtet ist, um die Freisetzung und/oder Abgabe der pharmakologisch wirksamen Verbindung noch besser zu kontrollieren.

10. Draht, Schlauch oder Katheter nach den Ansprüchen 1 bis 9, wobei die pharmakologisch wirksame Verbindung aus der aus Heparin, gerinnungshemmenden Faktoren, Cytostatika und Antibiotika bestehenden Gruppe ausgewählt wird.

11. Medizinische Vorrichtung fiir die kontrollierte Freisetzung und/oder am Wirkungsort stattfindende Abgabe pharmakologisch wirksamer Verbindungen, welche einen beschichteten Draht, Schlauch oder Katheter nach den Ansprüchen 1 bis 10 umfasst.

12. Verfahren zur Herstellung einer medizinischen Vorrichtung, welches das Aufbringen mindestens einer Schicht aus einer Zusammensetzung, die ein hydrophiles biologisch verträgliches Material nach den Ansprüchen 1 bis 11 und eine pharmakologisch wirksame Verbindung umfasst, auf wenigstens einen Teil eines Drahts; Schlauchs oder Katheters umfasst.

## Revendications

1. Fil métallique, tube ou cathéter revêtu d'au moins une couche d'une matière hydrophile biocompatible contenant au moins un composé pharmacologiquement actif, dans lequel la matière hydrophile biocompatible est une matière de revêtement polymère comprenant un monomère hydrophile qui peut participer à des réactions de polymérisation radicalaire et qui est soluble dans l'eau sous des conditions ambiantes, ledit monomère hydrophile étant N-vinylpyrrolidinone.

2. Fil métallique, tube ou cathéter selon la revendication 1, dans lequel le fil métallique, le tube ou le cathéter est réalisé à partir d'un métal, d'un alliage ou d'une matière polymère ou d'une combinaison de ceux-ci.

3. Fil métallique, tube ou cathéter selon la revendication 1 ou 2, dans lequel le fil métallique ou le tube est sous la forme d'une bobine ou d'une spirale.

4. Fil métallique, tube ou cathéter selon les revendications 1-3, dans lequel la matière hydrophile biocompatible est un copolymère d'un monomère réactif hydrophobe et d'un monomère réactif hydrophile.

5. Fil métallique, tube ou cathéter selon la revendication 4, dans lequel le rapport du composant hydrophile et du composant hydrophobe est réglé pour réguler la libération et/ou la délivrance régulée du composé pharmacologiquement actif.

6. Fil métallique, tube ou cathéter selon la revendication 4 ou 5, dans lequel le rapport du composant hydrophile et du composant hydrophobe de la matière hydrophile biocompatible est de 0,1 à 100.

7. Fil métallique, tube ou cathéter selon les revendications 4-6, dans lequel le composant hydrophobe est choisi à partir d'acrylates et de méthacrylates hydrophobes, de préférence le N-butyl-méthacrylate.

8. Fil métallique, tube ou cathéter selon les revendication 1-7, dans lequel la matière hydrophile biocompatible comprend de plus un agent de réticulation.

9. Fil métallique, tube ou cathéter selon les revendications 1-8, dans lequel la matière biocompatible est revêtue de plus d'une matière (partiellement) soluble ou gonflable pour réguler de plus la libération et/ou la délivrance du composé pharmacologiquement actif.

10. Fil métallique, tube ou cathéter selon les revendications 1-9, dans lequel le composé pharmacologiquement actif est choisi à partir du groupe de l'héparine, de facteurs anticoagulants, d'agent cytostatique et d'antibiotiques.

11. Dispositif médical pour la libération et/ou la délivrance (sur site) régulée de composés pharmacologiquement actifs comprenant un fil métallique, tube ou cathéter revêtu selon les revendications 1-10.

12. Procédé pour la préparation d'un dispositif médical consistant à appliquer au moins une couche de composition comprenant des matières hydrophiles biocompatibles selon la revendication 1-11 et un composé pharmacologiquement actif à au moins une partie d'un fil métallique, tube ou cathéter.
